# EUROPÄISCHE PATENTSCHRIFT

(11) **EP 1 570 786 B1**
(45) Veröffentlichungstag und Bekanntmachung des Hinweises auf die Patenterteilung: **20.09.2006**
(21) Anmeldenummer: 05101052.8
(22) Anmeldetag: 14.02.2005
(51) Int. Cl.: A61B 6/12, A61B 17/22

(54) **Vorrichtung zum Einstellen der Fokuslage des Therapiekopfes einer Lithotripsieanlage**
Apparatus for adjusting the focal position of the therapy head of a lithotripsy device
Dispositif d'ajustement de focalisation de la tête de traitement d'un dispositif de lithotripsie

(30) Priorität: 01.03.2004 DE 102004010466
(43) Veröffentlichungstag der Anmeldung: 07.09.2005
(73) Patentinhaber: SIEMENS AKTIENGESELLSCHAFT, 80333 München (DE)
(72) Erfinder: Köpping, Heiko, 91301, Forchheim (DE); Lanski, Markus, 90408, Nürnberg (DE); Mahler, Matthias, 91058, Erlangen (DE); Tauber, Herbert, 91058, Erlangen (DE)

(56) Entgegenhaltungen:
- DE-A1- 4 400 997
- DE-A1- 10 231 071
- DE-C1- 4 003 350

## Beschreibung

Die Erfindung betrifft eine Vorrichtung zum Einstellen der Fokuslage des Therapiekopfes einer Lithotripsieanlage. Eine solche Anlage umfasst ein Röntgensystem meist in Form eines Röntgen-C-Bogens. Das eine Röntgenstrahlquelle und einen Bildverstärker umfassende Röntgensystem dient zur Bildgebung während einer Steinbehandlung. Zur Einjustierung des Stoßwellenfokus auf einen zentralen, vom Röntgensystem aus unterschiedlichen Winkeln erfassbaren Punktes, im Falle eines Röntgen-C-Bogens, also dessen Isozentrum, ist eine Zielmarkierung am Bildverstärker des Röntgensystems erforderlich. Eine diesem Zweck dienende Vorrichtung umfasst eine Fadenkreuzscheibe, die mit Hilfe einer Befestigungseinrichtung am Bildverstärker fixiert ist. Die Zielmarkierung dient weiterhin zur Positionierung eines zu behandelnden Steines im Stoßwellenfokus. Eine Fadenkreuzscheibe besteht aus einem für Röntgenstrahlen durchlässigen Material. In ihrem Zentrum ist ein Ziel- bzw. Fadenkreuz aus einem im Bildverstärker abbildbarem Material angebracht. Im Fall von Lithotripsieanlagen mit integriertem Röntgensystem ist der Bildverstärker bereits werksseitig mit einer Fadenkreuzscheibe versehen, die unverstellbar am Bildverstärker befestigt ist.

Das Dokument DE 44 00 997 A1 offenbart eine solche Vorrichtung.

Mit Blick auf Anwender mit geringer Anzahl von Steinbehandlungen wurde in neuerer Zeit insbesondere aus Kostengründen die starre mechanische Kopplung zwischen Röntgen- und Lithotripsiesystem aufgegeben. Es werden mobile Lithotripsieanlagen eingesetzt, die einem beliebigen Röntgensystem über eine mechanische Schnittstelle beigeordnet werden können. Damit steht die Möglichkeit zur Verfügung, bereits vorhandene und für andere Zwecke eingesetzte Röntgensysteme auch für die Lithotripsie zu nutzen. Ein Problem dabei ist aber die bei anderweitig eingesetzten Röntgensystem fehlende Fadenkreuzscheibe am Bildverstärker.

Davon ausgehend besteht die Aufgabe der Erfindung darin, eine Vorrichtung der eingangs genannten Art vorzuschlagen, mit der unterschiedlich ausgestaltete Bildverstärker nachrüstbar sind.

Diese Aufgabe wird durch eine Vorrichtung nach Anspruch 1 gelöst. Danach erstrecken sich vom Rand der Fadenkreuzscheibe längenvariable Verbindungselemente weg, deren von der Fadenkreuzscheibe wegweisendes äußeres Ende mittel- oder unmittelbar am Bildverstärker fixierbar ist. Durch die längenvariablen Verbindungselemente ist eine Anpassung an Bildverstärker mit unterschiedlichen Durchmessern und damit eine Nachrüstung von praktischen beliebigen Röntgensystemen möglich.

Bei der Anbringung einer Fadenkreuzscheibe kann es beispielsweise aufgrund von Toleranzen erforderlich sein, dass eine Zentrierung der Scheibe vorgenommen werden muss. Bei einer solchen Zentrierung wird das Fadenkreuzzentrum mit dem Mittelpunkt der Eintrittsfläche des Bildverstärkers in Überdeckung gebracht. Oft stimmt auch der Mittelpunkt der Eintrittsfläche mit dem geometrischen Mittelpunkt des Bildverstärkergehäuses nicht überein. Bei einer bevorzugten Ausgestaltung ist daher vorgesehen, dass drei vorzugsweise gleichmäßig über den Umfang der Fadenkreuzscheibe verteilte Verbindungselemente vorgesehen sind. Diese erlauben eine einfache Verstellung der Fadenkreuzscheibe in beliebige Richtungen, so dass je nach den gegebenen Verhältnissen eine Zentrierung des Fadenkreuzes auf einfache Weise vorgenommen werden kann.

Bei einer ersten Vorrichtungsvariante ist das äußere Ende eines Verbindungselementes an einer am Außenumfang des Bildverstärkers angebrachten Haltevorrichtung fixiert. Eine mit geringem technischen Aufwand realisierbare Ausgestaltung sieht vor, dass jedem Verbindungselement ein am Außenumfang des Bildverstärkers fixierbares Haltelement zugeordnet ist. Die Halteelemente wiederum, sind auf ebenfalls technisch einfach zu realisierende Weise durch ein um den Außenumfang des Bildverstärkers gelegtes, die Halteelemente am Bildverstärker festklemmendes Spannband fixiert. Vorzugsweise sind die Halteelemente lösbar am Bildverstärker fixierbar. Dadurch ergibt sich auch bei einer festen Verbindung zwischen Fadenkreuzscheibe, Halteelementen und Verbindungselementen die Möglichkeit, bei Bedarf die Fadenkreuzscheibe vom Bildverstärker abzunehmen. Auf besonders einfache Weise, insbesondere ohne zur Hilfenahme von Werkzeugen gelingt dies, wenn ein Spannband aus elastischem Material verwendet wird. Denkbar ist natürlich auch, dass die Fadenkreuzscheibe lösbar an den Verbindungselementen fixiert ist.

Bei einem bevorzugten Ausführungsbeispiel wird die Längenvariabilität eines Verbindungselementes dadurch erreicht, dass dieses in mehrere durch Sollbruchstellen voneinander abgegrenzte Längsabschnitte unterteilt ist, wobei jeder Längsabschnitt ein Fixierelement aufweist, mit dem das Verbindungselement am Rand der Fadenkreuzscheibe fixierbar ist. Je nach Durchmesser eines umzurüstenden Bildverstärkers werden die Verbindungselemente durch Abbrechen eines oder mehrerer Längsabschnitte gekürzt. Die Ausbildung der Fixierelemente als eine einen Längsabschnitt durchsetzende Bohrung gewährleistet ein einfaches Anbringen des Verbindungselementes am Rand der Fadenkreuzscheibe, die an entsprechenden Stellen Bohrungen aufweist. Die mechanische Verbindung zwischen den genannten Teilen kann einfach durch Schrauben oder Nieten erfolgen. Eine Schnappverbindung zwischen dem äußeren Ende eines Verbindungselementes und einem Halteelement gewährleistet, dass die Fadenkreuzscheibe, zusammen mit den an ihr fixierten Verbindungselementen vom Bildverstärker abgenommen werden kann. Die einer Röntgenstrahlenquelle zugewandte Eintrittsfläche des Bildverstärkers ist dann völlig frei von Fremdelementen. Es verbleiben allenfalls Halteelemente am Außenumfang des Bildverstärkers.

Die erwähnten Verbindungselemente mit Sollbruchstellen gestatten eine stufenweise Anpassung an unterschiedliche Durchmesser von Bildverstärkern. Bei dieser Ausführungsform ist aber eine stufenlose Anpassung möglich, wenn zwischen dem äußeren Ende eines Verbindungselementes und einem Halteelement ein Justierteil angeordnet ist, mit dem der Abstand zwischen Verbindungs- und Haltelement in einem der Länge eines abbrechbaren Längsabschnitts entsprechenden Ausmaß veränderbar ist.

Bei einem weiteren bevorzugten Ausführungsbeispiel ist eine Längenvariabilität eines Verbindungselementes dadurch gewährleistet, dass dieses aus einem elastischen Material gefertigt ist, und dadurch in seiner Längsrichtung dehnbar ist. Vorzugsweise ist das Verbindungselement lösbar am Halteelement fixiert. Dadurch kann die Spannkraft und die Spannlänge des Verbindungselementes den jeweiligen Durchmesserverhältnissen angepasst werden.

Bei einer zweiten Vorrichtungsvariante ist es nicht erforderlich eine Haltevorrichtung am Außenumfang eines Bildverstärkers anzubringen. Die Fixierung der Fadenkreuzscheibe erfolgt vielmehr dadurch dass die äußeren Enden der Verbindungselemente hakenförmig ausgebildet sind, wobei die hakenförmigen Enden am Außenumfang des Bildverstärkers ansetzbar sind. Die zu einer sicheren Fixierung erforderliche Haltekraft wird dadurch erreicht, dass wenigsten ein hakenförmiges Ende in Richtung auf die Außenumfangsfläche des Bildverstärkers vorgespannt ist. Eine solche Vorspannung wird dadurch erreicht, dass am äußeren Ende eines Verbindungselementes ein mit der Außenumfangsfläche eines Bildverstärkers zusammenwirkender federbeaufschlagter Schenkel angelenkt ist. Durch einen solchen Federschenkel ist neben einer sicheren Fixierung auch eine gewisse Anpassung an unterschiedlich bemessene Bildverstärker möglich. In größerem Ausmaß wird dies bei einer bevorzugten Ausgestaltung jedoch dadurch erreicht, dass ein Verbindungselement einen, an der Fadenkreuzscheibe fixierten ersten Abschnitt und einen, vom hakenförmigen Ende gebildeten zweiten Abschnitt aufweist, wobei die beiden Abschnitte in unterschiedlichen Längspositionen aneinander fixierbar sind.

Die Erfindung wird nun anhand von in den Zeichnungen dargestellten Ausführungsbeispielen näher erläutert. Es zeigen:
Fig. 1 eine ein Röntgensystem umfassende Lithotripsieanlage,
Fig. 2 in perspektivischer Darstellung eine erste Variante einer an einem Bildverstärker fixierten Vorrichtung zur Einstellung des Fokuspunktes eines Stoßwellenkopfes,
Fig. 3 den Ausschnitt III aus Fig. 2,
Fig. 4 eine Abwandlung der ersten Vorrichtungsvariante in perspektivischer Darstellung,
Fig. 5 eine zweite Vorrichtungsvariante in perspektivischer Darstellung,
Fig. 6 ein Verbindungselement der Vorrichtung nach Fig. 5 in perspektivischer Darstellung,
Fig. 7 ein Teil des Verbindungselementes von Fig. 6 in perspektivischer Darstellung,
Fig. 8 ein Verbindungselement der Vorrichtung nach Fig. 5 mit federnd angelenktem Schenkel.

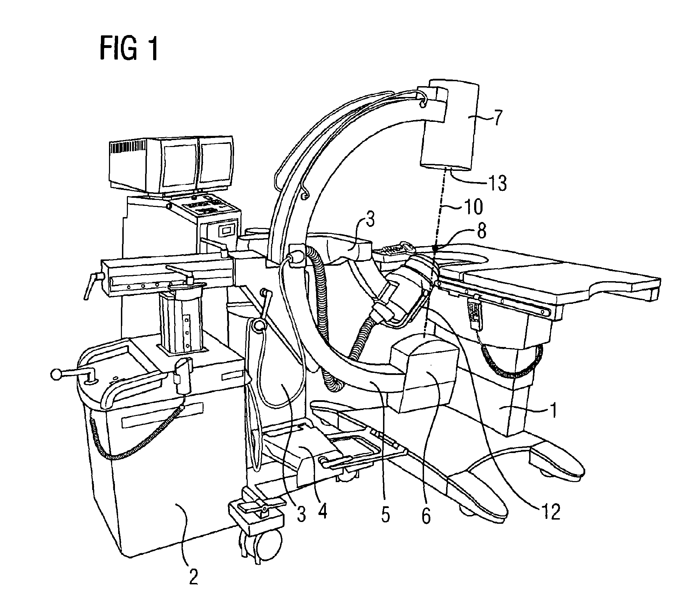

Fig. 1 zeigt eine Lithotripsieanlage, wie sie für Anwender mit geringerem Bedarf an Steinbehandlungen zweckmäßig ist. Sie umfasst einen mobilen Patiententisch 1, ein mobiles, auch für andere Zwecke einsetzbares Röntgen-C-Bogen-Gerät 2 sowie einen ebenfalls mobilen Lithotripter 3. Die genannten Baugruppen sind über eine gemeinsame mechanische Schnittstelle 4 aneinander fixiert. Das Röntgen-C-Bogen-Gerät 2 umfasst einen C-Bogen 5, dessen unteres Ende eine Röntgenstrahlenquelle 6 und dessen oberes Ende einen Bildverstärker 7 trägt. Vor der Durchführung einer Steinbehandlung ist der Fokuspunkt 8 des Therapiekopfes 9 des Lithotripters 3 so einzujustieren, dass er von einem Zentralstrahl 10 der Röntgenstrahlquelle 6 getroffen wird, wobei er zusätzlich im Isozentrum des Röntgen-C-Bogen-Gerätes 2 oder zumindest in dessen Nähe angeordnet ist. Zur Darstellung des Fokuspunktes ist am Therapiekopf 9 ein klappbarer Bügel 12 angeordnet, der eine im Röntgenbild sichtbare Metallkugel trägt. Zur Einjustierung des Fokuspunktes 8 ist eine Fadenkreuzscheibe14 (in Fig. 1 nicht dargestellt) erforderlich, die an der Eintrittsfläche 13 des Bildverstärkers 7 anzubringen ist. Bei ausschließlich für die Lithotripsie vorgesehenen Anlagen ist der Bildverstärker bereits werksseitig mit einer abnehmbaren Fadenkreuzscheibe ausgerüstet. Wird dagegen ein Röntgen-C-Bogen-Gerät 2 oder ein sonstiges Röntgensystem verwendet, das bisher für andere medizinische Zwecke eingesetzt wurde, ist eine Nachrüstung mit einer Fadenkreuzscheibe erforderlich. Eine eine solche Scheibe sowie Befestigungselemente umfassende Vorrichtung wird im folgenden beschrieben.

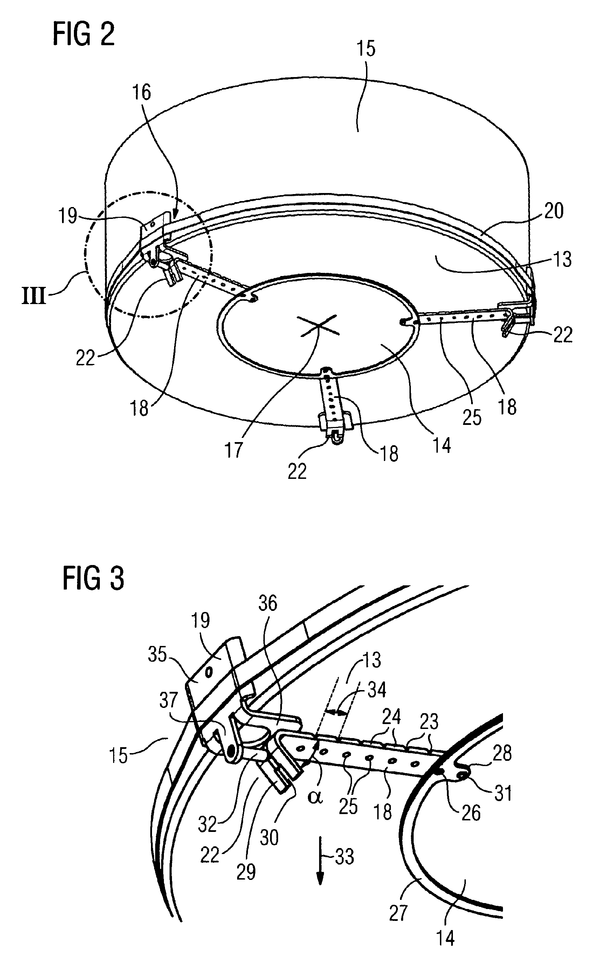

Fig. 2 bis 4 zeigen zwei Ausführungsbeispiele einer ersten Vorrichtungsvariante, welche eine an der Außenumfangsfläche 15 des Bildverstärkers 7 fixierbare Halteeinrichtung 16 umfasst. Im Montageszustand - auf den im folgenden stets Bezug genommen wird - liegt die Fadenkreuzscheibe 14 auf der Eintrittsfläche 13 im einjustierten Zustand so auf, dass das auf ihr vorhandene Fadenkreuz 17 den Eintrittspunkt des Zentralstrahles 10 bzw. das röntgenoptische Zentrum der Eintrittsfläche 13 markiert. Die Fadenkreuzscheibe 14 ist über insgesamt 3 gleichmäßig über den Umfang des Bildverstärkers 7 verteilte Verbindungselemente 18 mit der Halteeinrichtung 16 verbunden. Die Halteeinrichtung 16 umfasst drei mit einem Spannband 20 an der Außenumfangsfläche 15 festgeklemmte Halteelemente 19, mit denen die radial außen liegenden Enden 22 der Verbindungselemente 18 mechanisch gekoppelt sind. Die Verbindungselemente 18 sind streifenförmig ausgebildet und sind aus einem festen Material, etwa Aluminiumblech hergestellt. Bis auf die äußeren Enden 22 sind die Verbindungselemente 18 in mehrere durch quer verlaufende Kerben ausgebildete Sollbruchstellen 23 voneinander getrennte Längsabschnitte 24 unterteilt. Durch Abbrechen eines oder mehrerer Längsabschnitte 24 kann eine Anpassung an den jeweiligen Durchmesser des Bildverstärkers 7 erfolgen. Jeder Längsabschnitt 24 ist von einer Bohrung 25 durchsetzt, die zur Fixierung des Verbindungselements 18 an der Fadenkreuzscheibe dient. Diese weist einen versteiften Rand 27, bspw. ebenfalls aus einem Aluminiummaterial auf, in dem eine Bohrung 26 vorhanden ist. Die Befestigung des Verbindungselementes 18 erfolgt beispielsweise durch eine beide Bohrungen 22, 26 durchgreifende Schraube, (nicht dargestellt). Im Bereich eines Verbindungselementes 18 weist der Rand 27 einen radial nach innen gerichteten, von einer Bohrung 31 durchsetzten und zur Fixierung der Fadenkreuzscheibe 14 dienenden Vorsprung 28 auf.

Das äußere Ende 22 eines Verbindungselementes 18 ist hakenförmig umgebogen, wobei der Winkel α zwischen dem umgebogenen Abschnitt 29 und dem restlichen Verbindungselement kleiner 90° ist. Auf der radial nach außen weisenden Seite des Abschnittes 29 ist eine mittige, in Längsrichtung des Verbindungselementes 18 verlaufende Nut 30 vorhanden. In diese Nut greift ein am jeweiligen Halteelement 19 fixiertes Druckstück 32 mit einem in Eingriffsrichtung federbeaufschlagten Rastelement (nicht dargestellt) ein. Die Eingriffsstelle am Abschnitt 29 ist so positioniert, dass das Ende 22 des Verbindungselementes 18 mit einer zur Eintrittsfläche 13 gerichteten, aus der Schrägstellung des Abschnitts 29 resultierenden Kraft beaufschlagt wird. Zum Entfernen der Fadenkreuzscheibe 14 werden die Verbindungselemente in Richtung des Pfeiles 33 vom Bildverstärker abgezogen. Das Anbringen erfolgt in umgekehrter Richtung. Druckstück 32 und Abschnitt 29 wirken dabei im Sinne einer Schnappverbindung zusammen. Durch eine Stellschraube lässt sich das in die Nut 30 eingreifende federbeaufschlagte Raststück in Radialrichtung um einen Bereich verstellen, der etwa der Länge 34 eines Längsabschnittes 24 entspricht.

Die Halteelemente sind nach Art eines Winkelprofils ausgestaltete Blechteile, deren einer Schenkel 35 an der Außenumfangsfläche 15 und deren anderer Schenkel 36 an der Eintrittsfläche 13 des Bildverstärkers 7 anliegt. Vom Schenkel 35 steht eine diesen über die Eintrittsfläche 13 hinaus verlängernde Lasche 37 vor, die das Druckstück trägt. Die Schenkel 35 liegen mit umgebogenen Seitenrändern 41 linienförmig an der Außenumfangsfläche 15 an.

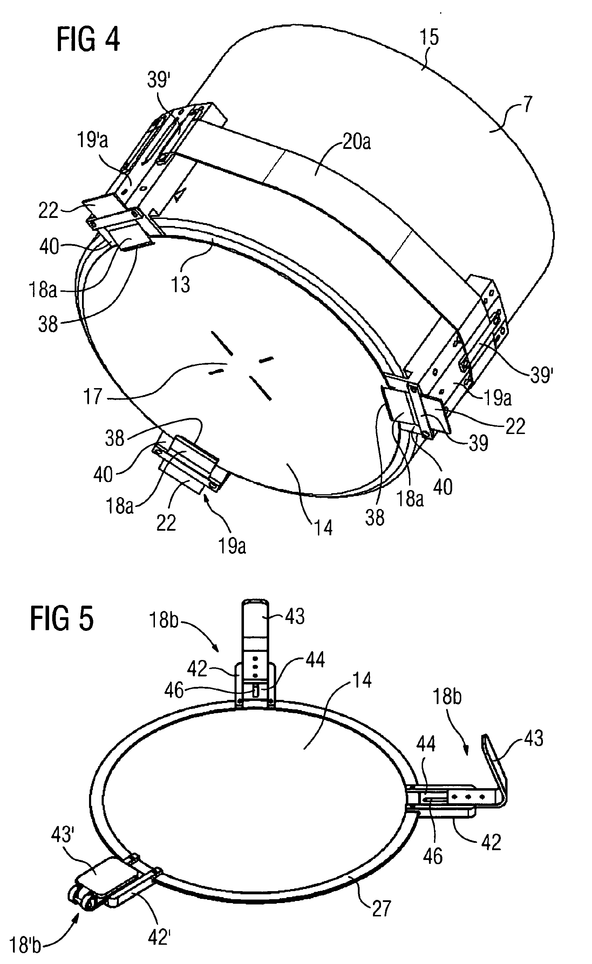

Figur 4 zeigt ein Ausführungsbeispiel, bei dem die Verbindungselemente 18a aus elastischem Material in Form eines Bandes gefertigt sind. Die Fixierung an einer Fadenkreuzscheibe 14 kann etwa dadurch erfolgen, dass diese einen Schlitz 38 aufweist, durch die das radial innere Ende des Verbindungselementes 18a unter Bildung einer Schlaufe hindurchgeführt ist. Das außenliegende Ende 22 ist an den Halteelementen 19a lösbar festgeklemmt. Die Klemmung erfolgt durch einen sich über das Verbindungselement 18a quer hinweg erstreckenden Steg 39, dessen überstehende Enden am Halteelement 19a festgeschraubt sind. Allein durch die Elastizität der Verbindungselemente 18a ist ein gewisser Spielraum bei der Anpassung an unterschiedlich bemessene Bildverstärker 7 vorhanden. Das Ausmaß der radialen Anpassungsfähigkeit kann noch dadurch vergrößert werden, dass die Verbindungselemente 18a an unterschiedlichen Längspositionen festgeklemmt werden. Durch Variation des Spannungszustandes bzw. der Klemmposition einzelner Verbindungselemente 18a ist auch eine Zentrierung des Fadenkreuzes 17 auf den Mittelpunkt der Eintrittsfläche 13 auf einfache Weise möglich. Die Halteelemente 19a sind durch ein elastisches Spannband 20a an der Außenumfangsfläche 15 des Bildverstärkers 7 festgeklemmt. Das Spannband ist mit seinen zwei Enden an der Außenseite eines Halteelementes 19'a mit Hilfe zweier Stege 39' festgeklemmt. Die übrigen Halteelemente 19a sind auf die gleiche Weise, nämlich ebenfalls mit einem Steg 39' am Spannband 20a fixiert. Um eine definierte Axiallage für die Halteelemente 19a zu schaffen, weisen diese an ihrer der Eintrittsfläche 13 zugewandeten Seite einen sich radial nach innen erstreckenden, plattenförmigen Anschlag 40 auf, der an der Eintrittsfläche 13 des Bildverstärkers 7 anliegt.

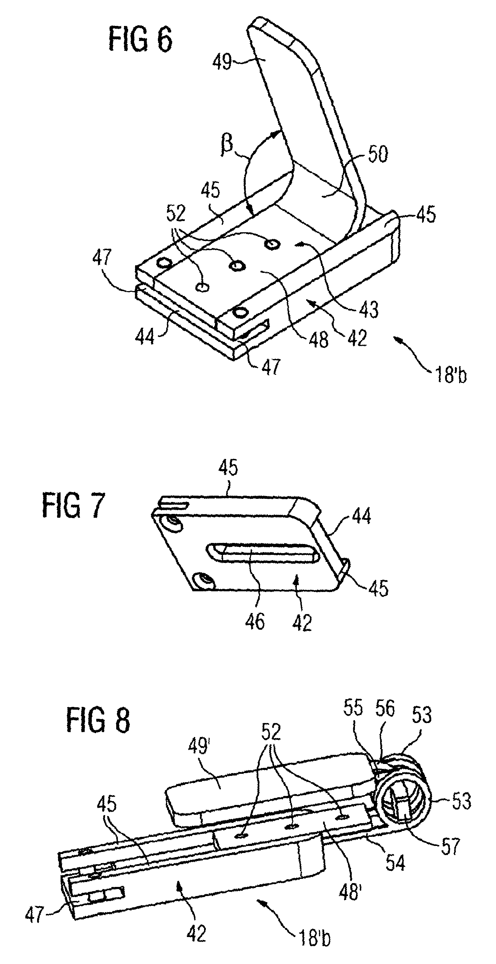

In Fig. 5 bis 7 ist eine Vorrichtungsvariante dargestellt, bei der die Fixierung an einem Bildverstärker 7 mit Hilfe der Verbindungselemente 18b selbst erfolgt. Die Verbindungselemente 18b sind aus einem ersten Abschnitt 42 und einem zweiten Abschnitt 43 gebildet. Der erste Abschnitt 42 ist ein im wesentlichen plattenförmiges Teil mit rechteckiger Umrissform. Auf seiner im Montagezustand der Eintrittsfläche 13 zugewandeten Seite ist eine sich über seine gesamte Länge erstreckende Ausnehmung 44 eingebracht. Die Breite der Ausnehmung 44 ist geringer als die Breite des ersten Abschnitts 42, so dass die Ausnehmung 44 von zwei seitlichen Randstreifen 45 flankiert ist. Der erste Abschnitt 42 ist weiterhin von einem mittig angeordneten, parallel zur Längserstreckung der Ausnehmung 44 verlaufenden Langloch 46 durchsetzt. In den der Fadenkreuzscheibe 14 zugewandten Stirnseiten des ersten Abschnitts 42 ist jeweils eine Ausnehmung 47 vorhanden, die den Rand 27 der Fadenkreuzscheibe 14 aufnimmt. Im Bereich der Ausnehmungen 47 weisen die Randstreifen 45 jeweils eine Bohrung 51 auf, mit der der Abschnitt 42 am Rand 27 fixierbar, etwa anschraubbar ist.

Der zweite Abschnitt 43 ist ein aus zwei Flachschenkeln gebildetes Winkelstück, wobei der erste Schenkel 48 bei seitlicher Führung durch die Randstreifen 45 längs verschiebbar in der Ausnehmung 44 einliegt und der zweite Schenkel 49 mit dem ersten Schenkel 48 einen Winkel β bildet, der kleiner als 90° ist. Der zweite Schenkel 49 liegt im Montagezustand an der Außenumfangsfläche 15 eines Bildverstärkers 7 an. Dadurch dass der von den beiden Schenkeln eingeschlossene Winkel β kleiner als 90° ist, liegt der zweite Schenkel 49 nur mit seinem Freiende am Bildverstärker an. Im Bereich der die beiden Schenkel 48, 49 verbindenden Hohlkehle 50 des zweiten Abschnitts 43 wird auf diese Weise ein Raum geschaffen, der beispielsweise einen die Eintrittsfläche umfassenden wulstartig verbreiterten Rand des Bildverstärkers aufnehmen kann. Der erste Schenkel 48 weist eine mittig angeordnete Reihe von Gewindebohrungen 52 auf, wobei zumindest in eine der Gewindebohrungen 52 eine das Langloch 46 durchgreifende Schraube (nicht dargestellt) mit ihrem Gewindeende eingedreht ist. Auf diese Weise ist der zweite Abschnitt 43 in unterschiedlichen Längspositionen am ersten Abschnitt 42 fixierbar und dadurch die Vorrichtung an unterschiedlich bemessene Bildverstärker anpassbar. Eines der drei Verbindungselemente 19b, nämlich das in Fig. 8 mit dem Bezugszeichen 18'b versehene, ist so ausgestaltet, dass es auf die Außenumfangsfläche 15 eines Bildverstärkers 7 eine Kraft ausübt und dabei die restlichen beiden Verbindungselemente 18b mit ihren beiden Schenkeln 49 an die Umfangsfläche 15 des Bildverstärkers 7 drückt. Die beiden Schenkel 48' und 49' des Verbindungselementes 18'b sind im wesentlichen ähnlich gestaltet, wie die entsprechenden Schenkel der Verbindungselemente 19b. Der zweite Schenkel 49' ist jedoch an dem ersten Schenkel 48' um eine parallel zu den Planebenen der Schenkel 48', 49' verlaufende Achse schwenkbar. Die beiden Schenkel 48', 49' sind über zwei koaxial nebeneinander angeordnete Schraubenfedern 53 verbunden. Die jeweils außen liegenden Federenden 54 sind mit dem Schenkel 48' und die jeweils innenliegenden Federenden 55 sind mit dem zweiten Schenkel 49' verbunden. Zwischen den beiden Schenkelfedern 53 ist ein Axialabstand vorhanden, in den ein stirnseitig am Schenkel 49' angeformter Fortsatz 56 hineinragt. Der Fortsatz 56 ist innerhalb der Schenkelfedern 53 zu einem sich in die Schraubenfedern 53 beidseitig hineinstreckenden Steg 57 ausgeformt. Der Steg 57 dient zur Lagefixierung bzw. als Schwenkachse bei einer Verschwenkung des Schenkels 49'.

Eine Vorrichtung bzw. ein Nachrüstsatz der beschriebenen Art kann mehrere Fadenkreuzscheiben 14 mit unterschiedlichen Durchmessern umfassen, wodurch sich zusätzlich zu der Längenvariabilität der Verbindungselemente 18 eine weitere Anpassung an Bildverstärker 7 unterschiedlicher Größen ergibt.

## Patentansprüche

1. Vorrichtung zum Einstellen der Fokuslage des Therapiekopfes (9) einer ein Röntgensystem umfassenden Lithotripsieanlage, mit einer an einem Bildverstärker (7) des Röntgensystems anbringbaren Fadenkreuzscheibe (14), **dadurch gekennzeichnet, dass** sich vom Rand der Fadenkreuzscheibe (14) längenvariable Verbindungselemente (18) wegerstrecken, deren von der Fadenkreuzscheibe (14) wegweisendes äußeres Ende (22) mittel- oder unmittelbar am Bildverstärker (7) fixierbar ist.

2. Vorrichtung nach Anspruch 1, **dadurch gekennzeichnet, dass** drei über den Umfang der Fadenkreuzscheibe (14) verteilte Verbindungselemente (18) vorhanden sind.

3. Vorrichtung nach Anspruch 1 oder 2, **dadurch gekenn- zeichnet, dass** das äußere Ende (22) eines Verbindungselements (18) an einer an der Außenumfangsfläche (15) des Bildverstärkers (7) angebrachten Halteeinrichtung (16) fixiert ist.

4. Vorrichtung nach Anspruch 3, **dadurch gekennzeich- net, dass** jedem Verbindungselement (18) ein an der Außenumfangsfläche (15) des Bildverstärkers (7) fixierbares Halteelement (19) zugeordnet ist.

5. Vorrichtung nach Anspruch 4, **gekennzeichnet durch** ein um die Außenumfangsfläche (15) des Bildverstärkers (7) legbare, die Halteelemente (19) am Bildverstärker (7) festklemmendes Spannband (20).

6. Vorrichtung nach Anspruch 4 oder 5, **dadurch gekenn- zeichnet, dass** die Halteelemente (19) lösbar am Bildverstärker (7) fixierbar sind.

7. Vorrichtung nach Anspruch 6, **gekennzeichnet durch** ein Spannband (20a) aus elastischem Material.

8. Vorrichtung nach einem der Ansprüche 1 bis 7, **dadurch gekennzeichnet, dass** die Fadenkreuzscheibe (14) lösbar an den Verbindungselementen (18) fixiert ist.

9. Vorrichtung nach einem der vorhergehenden Ansprüche, **dadurch gekennzeichnet, dass** ein Verbindungselement in mehrere von Sollbruchstellen voneinander abgegrenzte Längsabschnitte (24) unterteilt ist, wobei jeder Längsabschnitt (24) ein Fixierelement aufweist, mit dem das Verbindungselement (18) am Rand (27) der Fadenkreuzscheibe (14) fixiert ist.

10. Vorrichtung nach Anspruch 9, **dadurch gekenn- zeichnet, dass** das Fixierelement eine einen Längsabschnitt (24) durchsetzende Bohrung (25) ist.

11. Vorrichtung nach einem der Ansprüche 3 bis 10, **gekenn-zeichnet durch** eine Schnappverbindung zwischen dem äußeren Ende (22) eines Verbindungselements (18) und einem Halteelement (19).

12. Vorrichtung nach einem der Ansprüche 3 bis 11, **dadurch gekennzeichnet, dass** zwischen dem äußeren Ende (22) eines Verbindungselementes (18) und einem Halteelement ein Justierteil angeordnet ist, mit dem der Abstand zwischen Verbindungs- und Halteelement in einem der Länge eines Längsabschnitts (24) entsprechenden Ausmaß veränderbar ist.

13. Vorrichtung nach einem der Ansprüche 1 bis 8, **dadurch gekennzeichnet, dass** ein Verbindungselement (18a) aus einem elastischen Material besteht.

14. Vorrichtung nach Anspruch 13, **dadurch gekenn- zeichnet, dass** das äußere Ende (22) des Verbindungselements (18) lösbar am Halteelement (19) fixiert ist.

15. Vorrichtung nach Anspruch 1 oder 2, **dadurch ge- kennzeichnet, dass** das äußere Ende (22) der Verbindungselemente (18b) hakenförmig ausgebildet ist, wobei die hakenförmigen Enden an der Außenumfangsfläche (15) des Bildverstärkers (7) ansetzbar sind und dabei wenigstens ein hakenförmiges Ende in Richtung auf die Außenumfangsfläche (15) des Bildverstärkers (7) vorspannbar ist.

16. Vorrichtung nach Anspruch 15, **dadurch gekenn- zeichnet, dass** an einem vorspannbaren Ende ein mit der Außenumfangsfläche (15) zusammenwirkender federbeaufschlagter Schenkel (49') angelenkt ist.

17. Vorrichtung nach Anspruch 15 oder 16, **dadurch ge- kennzeichnet, dass** ein Verbindungselement (18b) einen an der Fadenkreuzscheibe (14) fixierten ersten Abschnitt (42) und einen, vom hakenförmigen Ende gebildeten zweiten Abschnitt (43) aufweist, wobei die beiden Abschnitte in unterschiedlichen Längspositionen aneinander fixierbar sind.

## Claims

1. Device for adjusting the focus position of the therapy head (9) of a lithotripsy system comprising an x-ray system, having a cross-hair disc (14) that can be affixed to an image intensifier (7), **characterised in that** variable-length connection elements (18) extend from the peripheral edge of the cross-hair disc (14), the outer end (22) pointing from the cross-hair disc (14) of which can be directly or indirectly affixed to the image intensifier (7).

2. Device according to claim 1, **characterised in that** three connection elements (18) distributed around the circumference of the cross-hair disc (14) are available.

3. Device according to claim 1 or 2, **characterised in that** the outer end (22) of a connection element (18) is affixed to a mounting device (16) attached to the outer circumference surface (15) of the image intensifier (7).

4. Device according to claim 3, **characterised in that** each connection element (18) is assigned a mounting element (19) which can be affixed to the outer circumference surface (15) of the image intensifier (7).

5. Device according to claim 4, **characterised by** a tightening strap (20) which can be placed around the outer circumference surface (15) of the image intensifier (7) and which clamps the mounting elements (19) to the image intensifier (7).

6. Device according to claim 4 or 5, **characterised in that** the mounting elements (19) can be fixed to the image intensifier (7) in a detachable manner.

7. Device according to claim 6, **characterised by** a tightening strap (20a) comprised of an elastic material.

8. Device according to one of claims 1 to 7, **characterised in that** the cross-hair disc (14) is affixed to the connection elements (18) in a detachable manner.

9. Device according to one of the preceding claims, **characterised in that** a connection element is subdivided into a number of length sections (24) demarcated from each other by predetermined breaking points, with each length section (24) comprising an attachment element allowing attachment of the connection element (18) at the edge (27) of the cross-hair disc (14).

10. Device according to claim 9, **characterised in that** the attachment element is a bore (25) penetrating through a length section (24).

11. Device according to one of claims 3 to 10, **characterised by** a snap connection between the outer end (22) of a connection element (18) and a mounting element (19).

12. Device according to one of claims 3 to 11, **characterised in that** an adjustment piece is arranged between the outer end (22) of a connection element (18) and a mounting element, with which adjustment piece the distance between the connection element and the mounting element can be changed to an extent corresponding to the length of the length section (24).

13. Device according to one of claims 1 to 8, **characterised in that** a connection element (18a) comprises an elastic material.

14. Device according to claim 13, **characterised in that** the outer end (22) of the connection element (18) is affixed to the mounting element (19) in a detachable manner.

15. Device according to claim 1 or 2, **characterised in that** the outer end (22) of the connection element (18b) is hook-shaped, with the hook-shaped ends being attachable to the outer circumference surface (15) of the image intensifier (7) and at least one hook-shaped end being pre-stressable here in the direction towards the outer circumference surface (15) of the image intensifier (7).

16. Device according to claim 15, **characterised in that** a spring-loaded leg (49') interacting with the outer circumference surface (15) is attached to a pre-stressable end.

17. Device according to claim 15 or 16, **characterised in that** a connection element (18b) comprises a first section (42) affixed to the cross-hair disc (14) and a second section (43) formed by said hook-shaped end, with the two sections being affixable to each other at different longitudinal positions.

## Revendications

1. Dispositif pour régler la position focale de la tête (9) de traitement d'une installation de lithotripsie comprenant un système à rayons X, avec un disque (14) à réticule pouvant être installé sur un amplificateur (7) de luminance du système à rayons X, **caractérisé en ce que** des éléments (18) de liaison de longueur variable partent du bord du disque (14) à réticule et leur extrémité (22) extérieure, opposée au disque (14) à réticule, peut être indirectement ou directement fixée à l'amplificateur (7) de luminance.

2. Dispositif suivant la revendication 1, **caractérisé en ce qu'**il est prévu trois éléments (18) de liaison répartis sur le pourtour du disque (14) à réticule.

3. Dispositif suivant la revendication 1 ou 2, **caractérisé en ce que** l'extrémité (22) extérieure d'un élément (18) de liaison est fixée à un équipement (16) de maintien qui est installé sur la surface (15) de pourtour extérieure de l'amplificateur (7) de luminance.

4. Dispositif suivant la revendication 3, **caractérisé en ce qu'**un élément (19) de maintien pouvant être fixé sur la surface (15) de pourtour extérieure de l'amplificateur (7) d' luminance est associé à chaque élément (18) de liaison.

5. Dispositif suivant la revendication 4, **caractérisé par** une bande (20) de serrage pouvant être posée autour de la surface (15) de pourtour extérieure de l'amplificateur (7) d' luminance et bloquant par serrage les éléments (19) de maintien sur l'amplificateur (7) de luminance.

6. Dispositif suivant la revendication 4 ou 5, **caractérisé en ce que** les éléments (19) de maintien peuvent être fixés de manière amovible sur l'amplificateur (7) de luminance.

7. Dispositif suivant la revendication 6, **caractérisé par** une bande (20a) de serrage en matériau élastique.

8. Dispositif suivant l'une des revendications 1 à 7, **caractérisé en ce que** le disque (14) à réticule est fixé de manière amovible aux éléments (18) de liaison.

9. Dispositif suivant l'une des revendications précédentes, **caractérisé en ce qu'**un élément de liaison est subdivisé en plusieurs parties (24) longitudinales délimitées les unes par rapport aux autres par des points de rupture privilégiée, sachant que chaque partie (24) longitudinale comporte un élément de fixation par lequel l'élément (18) de liaison est fixé sur le bord (27) du disque (14) à réticule.

10. Dispositif suivant la revendication 9, **caractérisé en ce que** l'élément de fixation est un perçage (25) traversant une partie (24) longitudinale.

11. Dispositif suivant l'une des revendications 3 à 10, **caractérisé par** une liaison à enclenchement entre l'extrémité (22) extérieure d'un élément (18) de liaison et un élément (19) de maintien.

12. Dispositif suivant l'une des revendications 3 à 11, **caractérisé en ce qu'**une pièce d'ajustement est disposée entre l'extrémité (22) extérieure d'un élément (18) de liaison et un élément de maintien, pièce qui permet de modifier la distance entre l'élément de liaison et l'élément de maintien dans une mesure correspondant à la longueur d'une partie (24) longitudinale.

13. Dispositif suivant l'une des revendications 1 à 8, **caractérisé en ce qu'**un élément (18a) de liaison est constitué d'un matériau élastique.

14. Dispositif suivant la revendication 13, **caractérisé en ce que** l'extrémité (22) extérieure de l'élément (18) de liaison est fixée de manière amovible à l'élément (19) de maintien.

15. Dispositif suivant la revendication 1 ou 2, **caractérisé en ce que** l'extrémité (22) extérieure de l'élément (18b) de liaison est réalisée en forme de crochet, sachant que les extrémités en forme de crochet peuvent être engagées sur la surface (15) de pourtour extérieure de l'amplificateur (7) de luminance et qu'au moins une extrémité en forme de crochet peut être précontrainte en direction de la surface (15) de pourtour extérieure de l'amplificateur (7) de luminance.

16. Dispositif suivant la revendication 15, **caractérisé en ce qu'**une branche (49') coopérant avec la surface (15) de pourtour extérieure est articulée à une extrémité pouvant être précontrainte.

17. Dispositif suivant la revendication 15 ou 16, **caractérisé en ce qu'**un élément (18b) de liaison comporte une première partie (42) fixée au disque (14) à réticule et une deuxième partie (43) constituée par l'extrémité en forme de crochet, sachant que les deux parties peuvent être fixées l'une à l'autre dans des positions longitudinales différentes.
